Europäisches Patentamt

European Patent Office

Office européen des brevets

(19)

(11) Publication number: **0 159 776**
**B1**

(12) **EUROPEAN PATENT SPECIFICATION**

(45) Date of publication of patent specification: **20.07.88**

(51) Int. Cl.⁴: **A 61 F 5/44**

(21) Application number: **85301088.2**

(22) Date of filing: **19.02.85**

(54) **Bladder assist device.**

(30) Priority: **23.02.84 JP 33981/84**
**06.04.84 JP 70525/84**

(43) Date of publication of application:
**30.10.85 Bulletin 85/44**

(45) Publication of the grant of the patent:
**20.07.88 Bulletin 88/29**

(84) Designated Contracting States:
**DE FR GB**

(56) References cited:
**DE-C- 514 765**
**US-A-3 604 420**
**US-A-4 084 593**

(73) Proprietor: **UNITIKA LTD.**
**50, Higashi Hommachi 1-chome**
**Amagasaki-shi Hyogo-ken (JP)**

(72) Inventor: **Mochizuki, Masatsugu**
**1965 Sugitani**
**Konan-cho Koga-gun Shiga-ken (JP)**
Inventor: **Jige, Masanobu**
**16 Biwa**
**Uji-Uji-shi Kyoto-fu (JP)**
Inventor: **Umemura, Yoshihiro**
**3-13 Kurumada**
**Todo Uji-shi Kyoto-fu (JP)**

(74) Representative: **Atkinson, Peter Birch et al**
**Marks & Clerk Scottish Life House Bridge Street**
**Manchester M3 3DP (GB)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

## Description

The present invention relates to a bladder assist device for maintaining natural drainage by repletion and contraction of the bladder, even when a urethral catheter is embedded in the bladder.

A patient suffering from spinal cord injury, encephalorrhagia or encephalomalacia, a patient after operation or a bedridden old man is liable to show symptoms of dysuria, urine incontinence and the like. In such a case, a urethral catheter has been widely used in view of ensuring a urinary pass and maintaining or improving the kidney function, or preventing a leaking of urine. However, in case of a continuous drainage method wherein the urethral catheter is indwelt in the body for a long term, the bladder is always contracted and therefore a normal drainage function obtained from a repetition of repletion and contraction is completely lost. Generally, man has the following drainage mechanism; storing urine in the bladder, stimulating an inherent receptor in the bladder wall with extension of the bladder wall from repletion with urine, reaching the stimulation to the drainage center of cerebrum from the myelon through the brain stem and then adversely returning from the cerebrum to the myelon, contracting the bladder wall, relaxing the muscle group of the pelvis bottom, relaxing the urethral sphincter muscle and urinating. On the other hand, the drainage mechanism is stopped after the insertion of the catheter in the patient having the catheter embedded in the body, because the most essential process in the drainage mechanism which is the repletion with urine and the stimulation of the receptor with the extension of the bladder wall is lost. In other ward, function as the bladder is stopped 100% and the bladder turns into a passage of urine or a collecting space of urine. In such a case, there are medical and physiological problems which have been discussed for a long time. The problems are summarized as the following three: the first is that, since a patient is laid on one's back and the back bottom of the bladder becomes a dead space to gather a little amount residual of urine, which, in turn, becomes a good culture ground for microbes invaded from inside or outside of the catheter tube, the bladder is changed into a hotbed for raising cystitis, pyelitis or pyelonephritis. The second is that, since a tip of the balloon catheter inserted into the bladder always presses a part of the contracted bladder wall, which results in an ulcer by a poor circulation of the blood and then a necrosis which is known as "Foley tip necrosis", the part of these diseases changes to a suitable entrance for microbes, which may cause cystitis to develop in high possibility. The third problem is that, since the bladder function is completely stopped by use of the catheter, recovery of the bladder function becomes more delayed when the use of the catheter is longer. In case of a patient suffering from ischuria after an operation in a pelvis, the spinal cord injury or a cerebrum blood vessel disease, it is pointed out that the patient having used the embedded catheter for a long term has difficulty in training the bladder, though such a training of the bladder is usually started in the subacute period (a recovering period of the drainage function).

As the solutions of the above problems associated with the long-term indwelling of the catheter, U.S. Patent No. 3,503,401 or 4,084,593 proposed a drainage control system and a bladder training apparatus in which outlets of the urethral catheter are connected to a siphon 3,503,401 or 4,084,593. Since these apparatus let the bladder to repeat repletion and contraction alternately when the urethral catheter is indwelt in the body, they seem to be useful for dissolving the above problems. However, they still have problems to be dissolved for a clinical use.

For example, the drainage control system described in U.S. Patent 3,503,401 is an open system in which a drainage tube connected to a collection container from the urethral catheter is opened to the air. In this system, a transient pressure reduction period in a drainage tube arises right after siphoning, whereby a back flow can be produced from the air to bladder. The back flow may be stronger than the conventional continuous drainage method with the indwelling urethral catheter and may contain the microbes associated with dusts in the air or the polluted urine in the form of bubbles. The invasion of microbes from the air to the bladder can happen in high possibility, which causes an infection by the microbes. The microbes having invaded in the bladder begin to proliferate in number like an exponential function, but the more desirable thing, of course, is to reduce the number of the microbes as few as possible. Even though this system considers dilution and discharge of the microbes invaded into the bladder, it is not satisfied with preventing the invasion of the microbes. This is the major reason not to provide clinical use. This problem is also present in the invasion of microbes from an air tube. The second problem is that a part forming the siphon tube is so thin and particularly so long that the liquid in the siphon tube can not be drained away completely and that plenty of liquid columns are closed up in the tube. In such a case, the siphon does not work well afterward. For example, if the air between two liquid columns has low pressure, the siphon begins to work before the bladder filling up with urine.

In the bladder training apparatus of U.S. Patent 4,084,593, the drainage cycle is very long in comparison with a usual drainage cycle. The intermittent auto-drainage method accomplished by the above apparatus is superior to the conventional continuous drainage method in view of an anti-infection effect because the intermittent process makes urine drained away completely within a time during which the invaded microbes does not proliferate sufficiently.

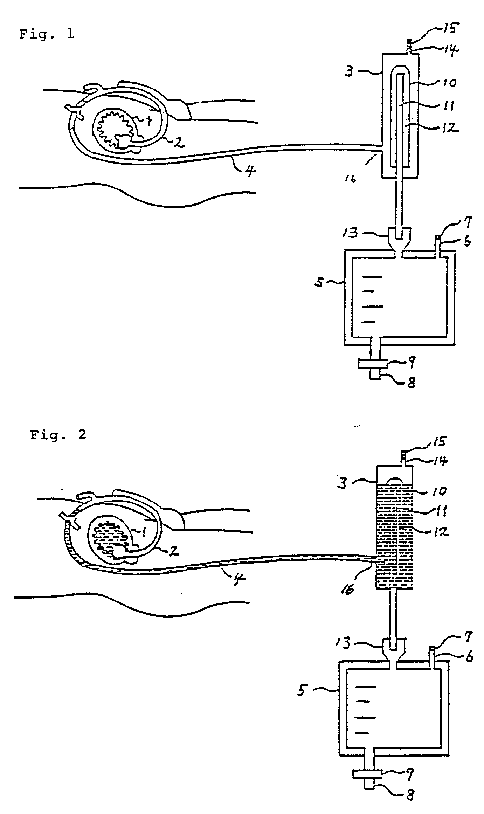

However, the microbes proliferate explosively to result in the cystitis when the drainage cycle is long enough to store urine for a long time. Particularly, in the intermittent drainage in which the bladder is temporarily filled up, pyelonephritis which is a serious ill can be developed. The problem hereinbefore is known to persons pertained in the medical field who are concerned in the urology, which is also described in Modern Medicine, by Nishiura, December, page 22 (1979). It has been found that intervals of drainage should be done preferably within 3 hours, at latest within 5 hours. However, in the bladder training apparatus of the above patient, a volume of a pressure chamber which is located below the bladder is bigger than that of the bladder and a lower end of the inverse U-shaped siphon tube in the chamber is deeply inserted into the chamber as shown in the specification, particularly in Fig. 1 and Fig. 2 (U.S. Patent 4,084,593 does not disclose the size of the apparatus excepting the inside diameter of the siphon tube, but it discloses that the relative size or location is as shown in the drawings). The amount of liquid to be drained by one siphoning, in other word, the amount of liquid to be filled before the next siphoning, is nearly equal to a total of the volume of the bladder $(V_1)$ and the volume of the above chamber above the lower end of the siphon tube $(V_2)$. The bigger $V_2$ makes the drain cycle more apart from the usual cycle and the intervals of drainage longer. From this point of view, it is desirable that the depth of the siphon tube of the above mentioned pressure chamber is as shallow as possible. However, if the depth of the siphon tube is allowed to be shallow, the waste fluid above the lower end of the siphon tube will be drained away and the siphoning will be stopped before whole content of the bladder is drained away because a large flow-pressure loss by the thin inside diameter of the catheter will occur. The above problem is difficult to dissolve for mutual intimate relations between $V_1$, $V_2$, the inside diameter of the catheter and the inside diameter of the siphon tube. This problem is dissolved for the first time by the present invention in which a pressure control hole sealed by a filter having air permeation restrain property is equipped with the pressure chamber, which is described hereinafter. On considering this point, a tube of the bladder training apparatus in U.S. Patent 4,084,593 corresponding to the air control tube of the present invention is connected to a collection container or an air-containing bag and therefore the air is free to flow, which can not solve the above problem. As described hereinbefore, the bladder training apparatus of U.S. Patent 4,084,593 is useful for a short term training of the bladder, but it is not good for a long term training, because it does not have a sufficient anti-infection property and therefore causes the infection fixed.

Also, U.S. Patent 2,602,448 or 2,860,636 disclose a tidal drainage and irrigating unit in which repletion and contraction of the bladder is alternately repeated according to the siphon principle. These apparatus are different in the following points.

Both apparatus are bladder irrigating units, in which the irrigating mechanism comprises repetition of the following cycle; an irrigating agent flowed down to the inside of the apparatus from the outside of the apparatus is flowed into the bladder from a tube connected to the bottom of the apparatus through a catheter, the inside pressure of the bladder is getting high and the liquid surface in the apparatus is getting high as the bladder is filling up with the agent and urine, and then the siphoning is begun to finish a drain of the content in the bladder. Different points between the apparatus of the above US Patent and the device of the present invention are not only that the apparatus has the inlet for the irrigating agent at the top of the apparatus, but also that a connection inlet to the catheter is located lower than the lower end of the inlet of the outer siphon tube. If the connection inlet is located above the lower end of the outer siphon tube, air flows into the bladder, which is shown by the operation mechanism of the apparatus of both of U.S. Patents. On the other hand, in the apparatus of the present invention, a connecting nozzle connected to the catheter should be located above the lower end of the inlet of the siphon tube from the following two points:

(1) no material flows into the bladder from the device.

(2) it is necessary that residual urine in the bladder should be decreased as little as possible after siphoning.

The present invention provides a bladder assist device comprising a urethral catheter adapted to be inserted in the bladder, a pressure control chamber which is connected either directly or through a connecting tube to the outlet of the urethral catheter at a location below the insertion end of the catheter, a urine collecting chamber located below the pressure control chamber, and an air inlet provided on the pressure control chamber for permitting air flow into the pressure control chamber, characterised in that the pressure control chamber contains a concentric-type siphon comprising an inner tube and a concentric outer tube, the inlet into the pressure control chamber being at a location between the upper end of the inner tube and the lower end of the outer tube, the upper end of the inner tube being located above the insertion end of the catheter; the urine collecting chamber is connected to the inner tube of the siphon; and the air inlet contains a filter having limited permeability to air so as to restrict the air flow rate through the air inlet.

Drawings show an embodiment of the bladder assist device of the present invention.

Fig. 1 is a whole view of the apparatus of the present invention.

Fig. 2 shows a situation that the bladder is repleted with urine before siphoning.

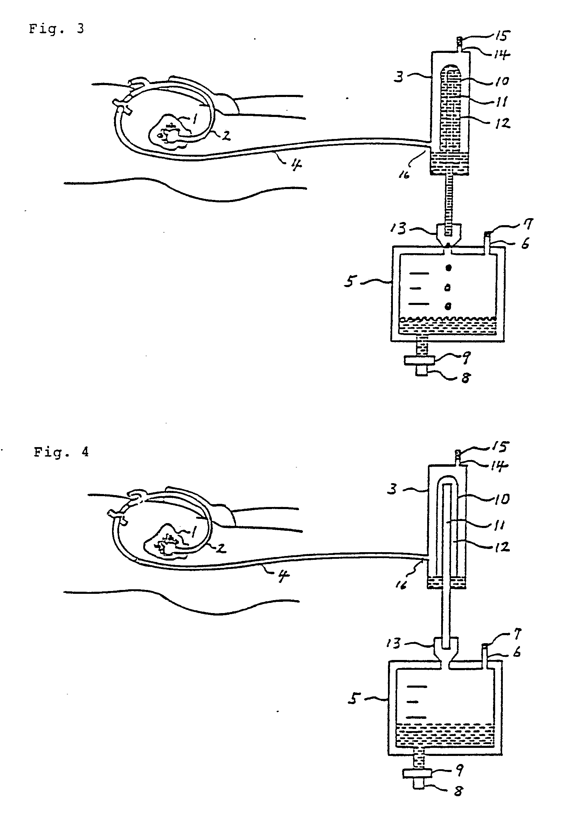

Fig. 3 shows a situation just before finishing the siphoning, when the bladder is already emptied.

Fig. 4 shows a situation that the siphoning is finished.

In the drawings, numbers indicates as follow; the bladder 1, the urethral catheter 2, the pressure control chamber 3, the tube 4 connecting the outlet of the urethral catheter 2 to the pressure control chamber 3, the urine collecting chamber 5 located below the pressure control chamber 3, a vent hole 6 equipped with the urine collecting chamber 5, a microporous filter for sterilization 7 equipped with the vent hole 6, an outlet 8 equipped with the urine collecting chamber 5, a stopper 9, the concentric-type siphon tube 10 contained in the pressure control chamber 3, the inner tube 11 of the concentric-type siphon tube 10, the outer tube 12 of the concentric-type siphon tube 10, a non-return device 13 of microbes equipped with the entrance part of the urine collecting chamber 5, the pressure control hole 14 located above the upper portion of the concentric-type siphon tube 10, the filter 15 having air permeation restrain property for sealing the pressure control hole 14 and a connecting nozzle 16.

Constructional features of the device of the present invention are the concentric-type siphon tube 10 for which the device becomes compact and can be easily treated in comparison with a conventional apparatus using an inverse U-shape siphon tube, and the pressure control hole 14 sealed by the filter 15 having air permeation restrain property and located above the upper portion of the concentric-type siphon tube 10 contained in the pressure control chamber 3. In the conventional apparatus, since the inside diameter of the urethral catheter is generally thin (2 to 3 mm) and long enough to have a large pressure loss when urine flows in the catheter, a supply rate $(Q_1)$ from the bladder 1 to the pressure control chamber 3 does not catch up with a drain rate $(Q_2)$ of the pressure control chamber 3 through the siphon tube 10 during siphoning, whereby the pressure control chamber is liable to become emptied before the bladder 1 is completely emptied. In the present invention, the pressure control hole 14 is sealed by the filter 15 having air permeation restrain property to reduce the siphoning rate and to enable to maintain a reduced pressure for a long time which arises on siphoning in the system. Accordingly, $Q_1$ endlessly approaches $Q_2$ for the first time. This means that it becomes possible that the removal volume $V_2$ of the pressure control chamber 3 (a volume between the lower end of the outer tube 12 of the concentric-type siphon tube 10 and the upper portion of the inner tube 11 of the concentric-type siphon tube 10) is reduced to the minimum, which makes the drainage cycle shortened and approached to an inherent drainage cycle. In other ward, the one merit by equipping the filter 15 having air permeation restrain property in the present invention is that the shortening of the drainage cycle makes it possible to drain urine away before the increase of the microbes, even when they are invaded into the bladder from the outside or inside of the catheter tube. Further, since this filter 15 also fulfils a role of a microbe-removing filter, a pollution by microbes from the outside of the system because of a reduced pressure in the system on siphoning can be prevented. Furthermore, the reduced pressure in the system which is risen from siphoning is maintained for a long time to reduce the distance between the upper end and the lower end of the concentric-type siphon tube, which is a working energy for flowing into the chamber from the bladder, which makes a miniaturization of the apparatus possible.

The air permeation restrained filter 15, preferably, has a flow rate of about 10 to about 20,000 ml/min. Flow rates less than 10 ml/min affect on siphoning, and preferred flow rates, therefore, are at least 10 ml/min, desirably at least 50 ml/min, particularly at least 100 ml/min. Flow rates more than 20,000 ml/min make the siphoning very fast so as to empty the pressure control chamber 3 before the bladder is emptied, and preferred flow rates, therefore, are not more than 20,000 ml/min, desirably not more than 10,000 ml/min, particularly not more than 5,000 ml/min, more particularly not more than 1,000 ml/min, the air flow rate means a value filtrated with non-particle air of 20°C under the pressure of 0.07 kg/cm² (1 PSI). Various kind of the air permeation restrained filter 15 can be prepared by limiting a pore size, a porosity and an area size. Preferred pore size is less than 1 micron in view of preventing the invasion of the microbes as few as possible. Materials and forms of the air permeation restrained filter 15 are not limited. Generally, membrane filter, sponge, unwoven fabric, cotton sealer and the like which prepared from regenerated cellulose, nitrocellulose, acetate cellulose, polyvinylidene fluoride, polytetrafluoroethylene and the like can be employed.

The air permeation restrained filter 15 can be equipped with the pressure control chamber 3 at a location above the upper portion of the concentric-type siphon tube 10 in order not to be polluted by urine which may go up to the upper portion of the siphon tube 10 in the pressure control chamber 3. Preferably, the filter 15 can be installed to the chamber 3 with a enough room in height, because a sudden rise of the liquid surface may be happened by a momental pressure change at an abdominal region when a patient had a cough or a sneeze. A barrier to prevent the sudden rise of the liquid may also be equipped with the filter.

The inner tube 11 of the concentric-type siphon tube 10, preferably, has an inside diameter of 1 to 10 mm, preferably 3 to 7 mm. Inside diameters less than 1 mm are not preferred because, after siphoning, short liquid columns can be closed up in the tube in high possibility, on the other hand, inside diameters more than 10 mm affect on siphoning. The outer tube 12 of the concentric-type siphon tube 10, preferably, has an inside diameter within such a range that a cylindrical sectional area between the outside wall of the inner tube and the inside wall of the outer tube is nearly equal to the inside sectional area of the inner tube 11. The lower end of the outer tube 12 of the concentric-type siphon tube 10, preferably, has such a construction as described in Fig. 4, Fig. 6, Fig. 7 and Fig. 8 of U.S. Patent 2,602,448. The volume of the pressure control chamber 3 is, preferably, from 50 to 150 ml, and the content volume $V_2$ between the lower end of the outer tube 12 and the upper portion of the inner tube 11 of the concentric-type siphon tube 10 in the pressure control chamber 3 is preferably less than 100 ml. A urine amount of a grown-up man is about 1,500 ml/day and, when the content ($V_1$) of the bladder is set to about 200 ml, the drain amount ($V_1$—$V_2$) by one siphoning process becomes about 200 to 300 ml, whereby drainage can be conducted 5 to 7 times a day, or once in 3 to 5 hours.

The content volume of the urine collecting container 5 is about 2,000 ml which is equal to the maximum urine amount of a patient per day. Preferred container 5 is a closed-type urine bag which is commercially available, comprising a microbes non-ascending device or drip chamber 11 at the entrance of the bag, a vent hole 6 having a microporous filter for sterilization 7, and a drain 8 to remove urine from the bottom of the bag.

As the urethral catheter 2 of the device of the present invention, a Foley balloon catheter suitable for embedding in the body for a long term can be preferably employed. Other examples of the catheter are a double balloon catheter and a different shape balloon catheter which are expanded at the prostate part in order to prevent a leakage of urine between the outside wall of the catheter and the urethral mucosa membrane. Also a three way balloon catheter equipped with an inlet of a disinfectant agent for irrigating the bladder can be employed, whereby the disinfectant agent can be continuously introduced into the bladder with a dropping to irrigate the bladder automatically without a complicated treatment of the prior art.

In the bladder assist device of the present invention, the relative location is further described in detail. When the urethral catheter 2 is embedded in the bladder, the height between the insertion end of the catheter 2 and the upper portion of the inner tube 11 of the concentric-type siphon tube 10 can be varied within the range of 0 to 100 mm according to usage, because 100 mm of water column is nearly equal with a pressure for filling up the bladder of the usual person. If the pressure is high than necessity in comparison with a necessary pressure, it compels a patient who suffers from the atonic nervous bladder to excessively expand the bladder wall and then to destroy the receptor. When the urethral catheter 2 is embedded in the bladder, the height difference between the insertion end of the catheter 2 and the connecting nozzle 16 of the pressure control chamber 3 is preferably from 50 to 200 mm, which is much smaller than a conventional one. The height difference between the lower portion of the outer tube 12 of the concentric-type siphon tube 10 in the chamber 3 and the entrance of the urine collecting chamber 5 is generally from 100 to 300 mm.

Materials used for the device of the present invention are not limited. Examples of the materials are an injection molding hard plastic such as polycarbonate, polyacrylonitrile-styrene copolymer; or a soft plastic having flexibility such as soft polyvinylchloride.

In the present invention, the urethral catheter 2 can be an antimicrobial agent gradually release urethral catheter. All urethral catheters which can release gradually the antimicrobial agent can be employed in the present invention. The urethral catheter is generally obtained from materials such as natural rubber and silicone rubber. The antimicrobial agent is combined with the rubbers either in the manufacturing process or in a post-handling after manufacturing. Also the agent may be coated to, absorbed into or physically combined with the rubbers. When the natural rubber is used, the urethral catheter can be manufactured from dip molding an antimicrobial latex composition comprising a biguanide compound or a salt thereof, an acridine compound or a salt thereof, a quaternary ammonium salt compound, or a cation-type antimicrobial agent of the other antibiotics in a natural rubber latex (Japanese Patent Application Ser. No. 104572/1983, 148454/1983 and 188942/1983 which are filed by the present applicant). When the silicone rubber is used, a catheter obtained from combining the above cation type antimicrobial agent with silicone rubber at a step of mastication, and extruction molding or compression molding (Japanese Patent Application Ser. No. 188940/1983) or a catheter obtained from forming an active coating layer on the inside or outside of the silicone rubber catheter which is commercially available, and setting an antibiotic through ionic bond (Japanese Pat. Publn. (unexamined) No. 195471/1982, EP Publn. (unexamined) No. 65884, or U.S. Ser. No. 382,734 (May 27, 1982) which are filed by the present applicant) can be also employed in the present invention.

Catheters formed from hydrophilic treating the surface of a natural rubber catheter or silicone rubber catheter, or from forming a hydrophilic coating layer, and dipping an antimicrobial agent solution containing isozin and the like to absorb the antimicrobial agent physically can be used.

The present device provides the constant repetition of repletion and contraction of the bladder at a constant period without using any complicated machines, electric powers or electric control systems even when the catheter is embedded in the bladder, whereby a maintenance of the bladder function or a recovery training can be easily conducted in comparison with the conventional continuous drainage method and the urinary tract infection can be remarkably reduced. The present invention has the following technical effects:

(1) To reduce remarkably the number of the microbes in the bladder invading from the outside by using the antimicrobial agent gradually release urethral catheter,

(2) to prevent the storage of residual urine and the increase of microbes as brings into the spreading the cystitis by using a compelled irrigation or rinse treatment at a constant period (at latest for 5 hours, preferably within 3 hours),

(3) to enable to prevent an outbreak of the cystitis produced by a press necrosis of the bladder wall because of relieving from what we call "Foley tip necrosis" which occurs from pressing the contracted bladder wall with the pointed end of the catheter,

(4) to prevent atrophy of the bladder wall muscle which is observed in the contracted bladder and to increase resistance against infections which is observed in a normal bladder, and

(5) to reduce the risk of the invasion of the microbes through the catheter tube from outside, because the process of the repletion of the bladder prevents a reduced pressure which is always seen in case of the conventional continuous drainage method.

The device of the present invention is effective on a patient suffering from spinal cord injury, encephalorrhagia, encephalomalacia, or drainage trouble or urine incontinence of a patient after operation or a bedridden old man. In case of a patient suffering from a neurogenic bladder from the spinal injury, the indwelling of the catheter took for one to 2 months in the conventional continuous drainage method, but the present invention makes the period of indwelling of the catheter shortened to about 2 weeks without urethral infection when the training of the bladder is begun within the acute period to the subacute period, which has a significant meaning on a rehabilitation. When the device of the present invention was used for a bedridden old man, diseases such as the cystitis or fever was not developed after 2 months later and the bedridden old man was free from unpleasant conditions such as diaper rash.

### Claims

1. A bladder assist device comprising a urethral catheter (2) adapted to be inserted in the bladder, a pressure control chamber (3) which is connected either directly or through a connecting tube (4) to the outlet of the urethral catheter at a location below the insertion end of the catheter, a urine collecting chamber (5) located below the pressure control chamber, and an air inlet (14) provided on the pressure control chamber for permitting air flow into the pressure control chamber, characterised in that the pressure control chamber contains a concentric-type siphon (10) comprising an inner tube (11) and a concentric outer tube (12), the inlet into the pressure control chamber being at a location between the upper end of the inner tube and the lower end of the outer tube, the upper end of the inner tube being located above the insertion end of the catheter; the urine collecting chamber is connected to the inner tube of the siphon; and the air inlet contains a filter (15) having limited permeability to air so as to restrict the air flow rate through the air inlet.

2. The device of claim 1 in which the air permeation restrained filter 15 has a flow rate of about 10 to about 20,000 ml/min.

3. The device of claim 1 in which the inner tube 11 of the concentric-type siphon tube 10 has an inside diameter of 1 to 10 mm.

4. The device of claim 1 in which the inner tube 11 of the concentric-type siphon tube 10 has an inside diameter of 3 to 7 mm.

5. The device of claim 1 in which the height between the insertion end of the catheter 2 and the upper portion of the inner tube 11 of the concentric-type siphon tube 10 is from 0 to 100 mm.

6. The device of claim 1 in which the height between the insertion end of the catheter 2 and the connecting nozzle 16 of the pressure control chamber 3 is from 50 to 200 mm.

7. The device of claim 1 in which the urethral catheter is an antimicrobial agent gradually release urethral catheter.

### Patentansprüche

1. Blasenunterstützungsgerät, umfassend einen Urethralkatheter (2), der dazu geeignet ist, in die Blase eingeführt zu werden, eine Drucksteuerkammer (3), die entweder direkt oder durch ein Verbindungsrohr (4) mit dem Auslaß des Urethralkatheters an einer Stelle unterhalb des Einführungsendes des Katheters verbunden ist, eine Urinsammelkammer (5), die sich unterhalb der Drucksteuerkammer befindet, und einen Lufteinlaß (14), der zum Ermöglichen einer Luftströmung in die Drucksteuerkammer auf der Drucksteuerkammer vorgesehen ist, dadurch gekennzeichnet, daß die Drucksteuerkammer einen Sifon (10) vom konzentrischen Typ enthält, der ein inneres Rohr (11) und ein konzentrisches äußeres Rohr (12) umfaßt, wobei der Einlaß in die Drucksteuerkammer an einer Stelle zwischen dem oberen Ende des inneren Rohrs und dem unteren Ende des äußeren Rohrs ist, wobei sich das obere Ende des inneren Rohrs oberhalb des Einführungsendes des Katheters befindet; die Urinsammelkammer mit dem inneren Rohr des Sifons verbunden ist; und der Lufteinlaß ein Filter (15) enthält, das eine beschränkte Permeabilität für Luft hat, so daß es die Luftströmungsrate durch den Lufteinlaß beschränkt.

2. Gerät nach Anspruch 1, dadurch gekennzeichnet, daß das das Eindringen von Luft beschränkende Filter (15) eine Strömungsrate von etwa 10 bis etwa 20.000 ml/min hat.

3. Gerät nach Anspruch 1, dadurch gekennzeichnet, daß das innere Rohr (11) von dem Rohr (10) des Sifons vom konzentrischen Typ einen Innendurchmesser von 1 bis 10 mm hat.

4. Gerät nach Anspruch 1, dadurch gekennzeichnet, daß das innere Rohr (11) von dem Rohr (10) des Sifons vom konzentrischen Typ einen Innendurchmesser von 3 bis 7 mm hat.

5. Gerät nach Anspruch 1, dadurch gekennzeichnet, daß die Höhe zwischen dem Einführungsende des Katheters (2) und dem oberen Teil des inneren Rohrs (11) von dem Rohr (10) des Sifons vom konzentrischen Typ von 0 bis 100 mm ist.

6. Gerät nach Anspruch 1, dadurch gekennzeichnet, daß die Höhe zwischen dem Einführungsende des Katheters (2) und der Verbindungsdüse (16) der Drucksteuerkammer (3) von 50 bis 200 mm ist.

7. Gerät nach Anspruch 1, dadurch gekennzeichnet, daß das Urethralkatheter ein antimikrobischen Wirkstoff allmählich freisetzendes Urethralkatheter ist.

**Revendications**

1. Dispositif d'assistance vésicale comprenant une sonde urétrale (2) adaptée pour être introduite dans la vessie, une chambre de réglage de la pression (3) qui est raccordée soit directement soit par un tube de raccord (4) à la sortie de la sonde urétrale en un point situé au-dessous de l'extrémité d'insertion de la sonde, un réservoir de recueil d'urine (5) disposé au-dessous de la chambre de réglage de la pression et une admission d'aire (14) prévue sur la chambre de réglage de la pression pour permettre l'arrivée d'air dans la chambre de réglage de la pression, caractérisé en ce que la chambre de réglage de la pression contient un siphon de type concentrique (10) comprenant un tube intérieur (11) et un tube extérieur concentrique (12), l'entrée dans la chambre de réglage de la pression étant située en un point entre l'extrémité supérieure du tube intérieur et l'extrémité inférieure du tube extérieur, l'extrémité supérieure du tube intérieur étant disposée au-dessus de l'extrémité d'insertion de la sonde; la réservoir de recueil d'urine est raccordé au tube intérieur du siphon; et l'admission d'air contient un filtre (15) ayant une perméabilitée limitée à l'air afin de limiter le débit d'air passant par l'admission d'air.

2. Dispositif de la revendication 1 dans lequel le débit de passage d'air, à travers le filtre à perméabilité à l'air limitée, peut aller d'environ 10 à environ 20.000 ml/min.

3. Dispositif de la revendication 1 dans lequel le tube intérieur 11 du tube siphon de type concentrique 10 a un diamètre intérieur de 1 à 10 mm.

4. Dispositif de la revendication 1 dans lequel le tube intérieur 11 du tube siphon de type concentrique 10 a un diamètre intérieur de 3 à 7 mm.

5. Dispositif de la revendication 1 dans lequel le hauteur entre l'extrémité d'insertion de la sonde 2 et la partie supérieure du tube intérieur 11 du tube siphon de type concentrique 10 est de 0 à 100 mm.

6. Dispositif de la revendication 1 dans lequel la hauteur entre l'extrémité d'insertion de la sonde 2 et la buse de raccordement 16 de la chambre de réglage de la pression 3 est de 50 à 200 mm.

7. Dispositif de la revendication 1 dans lequel la sonde urétrale est une sonde urétrale du type à libération progressive d'un agent antimicrobien.

Fig. 1

Fig. 2

Fig. 3

Fig. 4